**Europäisches Patentamt**

**European Patent Office**

**Office européen des brevets**

(19)

(11) Veröffentlichungsnummer: **0 001 761**
**B1**

(12) # EUROPÄISCHE PATENTSCHRIFT

(21) Anmeldenummer: **78101096.2**

(22) Anmeldetag: **07.10.78**

(51) Int. Cl.³: **C 07 D 30 7/08**

(54) **Verfahren zur Reinigung von Tetrahydrofuran**

(30) Priorität: **31.10.77 DE 2748788**

(43) Veröffentlichungstag der Anmeldung:
**16.05.79 Patentblatt 79/10**

(45) Bekanntmachung des Hinweises auf die Patenterteilung:
**01.10.80 Patentblatt 80/20**

(84) Benannte Vertragsstaaten:
**BE DE FR GB NL**

(56) Entgegenhaltungen:
**DE - A - 1 693 053**
**DE - B - 1 272 293**
**DD - A - 31 929**
**US - A - 3 935 252**
**US - A - 3 980 672**

**Chemical Abstracts, vol. 81, no. 21,**
**25.11.74, Columbus, Ohio, USA**
**Abstract Nr. 135 933z**

**Beilsteins Handbuch der organischen Chemie,**
**Band 17, 1.Teil, 4.Auflage, 3.und 4.**
**Ergänzungswerk, 1974,**
**Springer-Verlag Berlin, Heidelberg,**
**New York, Seite 25**

(73) Patentinhaber: **BASF Aktiengesellschaft**
**Carl-Bosch-Strasse 38**
**D - 6700 Ludwigshafen (DE)**

(72) Erfinder: **Mueller, Herbert, Dr.**
**Carostrasse 53**
**D - 6710 Frankenthal (DE)**
**Huchler, Otto Hermann, Dr.**
**Weinbietstrasse 38**
**D - 6703 Limburgerhof (DE)**

Courier Press, Leamington Spa, England.

Verfahren zur Reinigung von Tetrahydrofuran

Tetrahydrofuran ist ein wichtiges Lösungsmittel und Zwischenprodukt für die chemische Industrie.

An die Reinheit von Tetrahydrofuran (THF) werden je nach Anwendungszweck mehr oder minder hohe Anforderungen gestellt. Handelsübliches Tetrahydrofuran technischer Qualität besitzt bereits einen sehr hohen Reinheitsgrad; normalerweise beträgt der Reingehalt über 99,8%. Selbst Verunreinigungen in einer Konzentration von z.B. 10 bis 500 ppm können aber für anspruchsvolle Anwendungen bereits schädlich sein.

Oft bilden sich derartige Verunreinigungen auch erst nachträglich beim Lagern und Transportieren des Lösungsmittels. So z.B. bildet Tetrahydrofuran sehr leicht Peroxide. Wird das Produkt nicht unter vollständigem Luftausschluß gehandhabt, so ist die Peroxid-Bildung unvermeidlich. Der Zerfall der Peroxide führt dann zur weiteren Verunreinigung durch Carbonylverbindungen.

Die genaue chemische Struktur aller im THF vorhandenen Nebenprodukte ist unbekannt, i.a. auch nicht wissenswert. Der Grad der Verunreinigung wird nämlich in der Praxis außer durch gaschromatographische Analyse im allgemeinen durch Bestimmung von Kennzahlen ermittelt. Dabei ist letztere Methode die für praktische Zwecke zuverlässigere: Die Bestimmung der Bromzahl nach Kaufmann, die Bestimmung der Carbonylzahl nach der Hydroxylaminhydrochlorid-Methode und die Peroxid-Bestimmung, die photometrisch mit Titanylsulfat durchgeführt wird, liefern die wichtigsten Kennzahlen.

Aufgabe der Erfindung ist es, ein Verfahren anzugeben, um technich reines Tetrahydrofuran auf einfache und billige Weise zu reinigen. Das Verfahren sollte anwendbar sein, gleichgültig, ob die Verunreinigungen bereits bei der Synthese oder nachträglich, beispielsweise durch Peroxid-Bildung entstanden sind.

An sich sind bereits eine Reihe von Verfahren bekannt geworden, um Tetrahydrofuran zu reinigen. So z.B. wird in der US—PS 3 980 672 vorgeschlagen, Tetrahydrofuran, das speziell durch n-Butyraldehyd verunreinigt ist, durch die Behandlung mit einem Molekularsieb einer Porengröße von 0,4 bis 0,5 nm und einer Wasseradsorptionswärme von 1 800±100 btu/pound $H_2O$ (= 4183.10³ ± 200.10³)/kg $H_2O$) zu reinigen. Diese auf eine spezifische Verunreinigung abgestimmte Reinigungsmethode ist ungeeignet, gleichzeitig die Carbonylzahl, die Bromzahl und den Peroxid-Gehalt herabzusetzen. Das gleiche gilt für die japanische Patentanmeldung 74—005 338, in der beschrieben wird, daß Methanol aus Tetrahydrofuran durch Molekularsiebe entfernt werden kann. Nach der japanischen Patentanmeldung 74—076 861 wird Acetaldehyd durch festes Kaliumhydroxid aus Tetrahydrofuran entfernt. Die japanische Patentanmeldung 73—075 563 beschreibt die Entfernung von Aldehyden durch die Behandlung mit Ammoniumsulfat. Keines dieser bekannt gewordenen Verfahren erreicht eine Verbesserung gleichzeitig der Brom-, Carbonyl- und der Peroxid Zahl. Sie wiesen außerdem den Nachteil auf, daß sie sehr viel bzw. teurere Reinigungsmittel verwenden müssen.

Es wurde nun gefunden, daß Tetrahydrofuran durch einfaches Behandeln mit Bleicherde so gereinigt werden kann, daß die Carbonylzahl, die Bromzahl sowie der Peroxid-Gehalt sich dem Wert 0 nähern. Die Bleicherden oder auch Fullererden gehören kristallographisch zu den Montmorilloniten. Es handelt sich um kolloidale, wasserhaltige Aluminiumhydrosilikate, in denen Aluminiumonen teilweise durch Eisenoder Magnesiumonen ersetzt sein können. Das Verhältnis von Kieselsäure zu den Oxiden zwei- bzw. dreiwertiger Metalle in diesen Mineralien beträgt etwa 4:1. Es sind handelsübliche Produkte, die meistens durch Säurebehandlung aktiviert werden bzw. in aktivierter Form angeboten werden und in großem Umfang zur Raffination von Speiseölen Fetten und Mineralölen verwendet werden.

Handelsübliche Bleicherden enthalten normalerweise 4 bis 8% Wasser. In dieser Form werden sie auch als Katalysatoren oder Adsorptionsmittel verwendet. Ein weiteres Merkmal der Erfindung ist, daß im Faller der Reinigung von Tetrahydrofuran die wirdsamste Reinigung mit solchen Bleicherden erzielt wird, die weniger als 3, vorzugsweise 1% Wasser oder weniger enthalten.

Die Herstellung entwässerter Bleicherden bereitet keine Schwierigkeiten. Durch Erhitzen auf 110 bis 150°C während 1 bis 8 Stunden gelingt es, den Wassergehalt der Erden unter 0,1% zu erniedrigen.

Die erfindungsgemäße Behandlung ist einfach und ohne große Kosten durchführbar. Da nicht bekannt ist, in welcher Weise die Behandlung wirkt und welche Spurenverunreinigungen im einzelnen entfernt werden, empfiehlt es sich häufig, Tetrahydrofuran generell vor der Verwendung der Behandlung zu unterwerfen. Ohne die Vorbehandlung können bei empfindlichen Anwendungen Fehlergebnisse auftreten.

Zur erfindungsgemäßen Behandlung von Tetrahydrofuran werden nur geringe Mengen Bleicherde benötigt. Vorteilhafte Ergebnisse werden schon erzielt, wenn die Bleicherde in Mengen von 0,1 bis 5, vorzugsweise von 0,5 bis 3 Gew.%, bezogen auf THF, angewendet wird. Natürlich kann THF auch mit größeren Mengen behandelt werden, doch bringt dies keinen weiteren Vorteil. Vielfach — dies ist auch in den Beispielen beschrieben — kann die Behandlung

mehrfach mit derselben Bleicherdemenge wiederholt werden, bevor diese erschöpft ist.

Zur Durchführung der Behandlung wird THF mit der Bleicherde innig gemischt. Die Einwirkungsdauer kann je nach Menge und Temperatur 3 Minuten bis 12 Stunden betragen. Vorzugsweise begnügt man sich mit Einwirkungszeiten von 0,5 bis 3 Stunden. Längere Einwirkung schadet nicht.

Da Bleicherden normalerweise als feine Pulver oder als feinkörniges Granulat vorliegen, wird man sie meist in suspendierter Form anwenden. Grundsätzlich ist ·es auch möglich, Granulat als fest angeordnetes Bett einzusetzen, über das das zu reinigende Tetrahydrofuran geführt wird.

Die Behandlung kann bei Raumtemperatur, vorzugsweise aber bei erhöhter Temperatur, z.B. beim Siedepunkt von Tetrahydrofuran, durchgeführt werden. Vorzugsweise angewandt werden Temperaturen zwischen 30 und 66°C.

Nach der Einwirkung der Bleicherde wirde diese von der Flüssigkeit in üblicher Weise abgetrennt. Hierzu eignen sich die üblichen physikalischen Trennmethoden. Es genügt z.B., den Feststoff durch Filtration oder Zentrifugieren abzutrennen. Eine andere einfache Trennmethode ist das Abdestillieren. Die Behandlung kann fortlaufend oder absatzweise vorgenommen werden. Fortlaufende Arbeitsweise empfiehlt sich besonders dann, wenn ein Festbett zum Reinigen verwendet wird. Wendet man für die Abtrennung der Flüssigkeit vom Feststoff die Destillation an, so kann das im Destillationssumpf zurückbleibende Reinigungsmittel i.a. wieder verwendet werden, bis seine Kapazität erschöpft ist. Eine Regenerierung ist i.a. nicht lohnend; sie kann aber gegebenenfalls durch Erhitzen auf 150 bis 200°C oder Extraktion mit einem geeigneten Lösungsmittel erfolgen.

### Beispiel

1 000 Gewichtsteile Tetrahydrofuran technischer Qualität werden mit 20 Teilen handelsüblicher Bleicherde, erhältlich unter der Bezeichnung Tonsil Optimum FF® (Süd-Chemie AG, München), das bis auf einen Wassergehalt von weniger als 0,1% entwässert war, während 2 Stunden bei 64°C gerührt. Ohne zu fraktionieren wird dann die flüssige Phase von der festen Phase durch Destillation abgetrennt. Der im Destillationssumpf verbleibende Rückstand — es handelt sich gewichtsmäßig um die Menge eingesetztes Tonsil — wird für weitere 5 Reinigungsgänge verwendet. Wie die nachfolgende Tabelle ausweist, ist die Reinigungskapazität der Bleicherde danach noch nicht erschöpft.

Ähnliche Ergebnisse wurden auch erhalten, wenn der Feststoff nich durch Destillation, sondern durch Filtration abgetrennt.wurde oder wenn als Bleicherde ein anderes Handelsprodukte, z.B. "Katalysator KSF" (Süd-Chemie AG, München) in derselben Konzentration angewendet wurde.

Bei den in diesem Beispiel verwendeten Handelsprodukten "Tonsil Optimum FF" und "Katalysator KSF" handelt es sich um kolloidale wasserhaltige Aluminium hydrosilikate, in denen die Aluminiumionen teilweise durch Eisen — oder Magnesiumionen ersetzt sind. Das Verhältnis von Kieselsäure zu den Oxiden der 2 — bzw. 3 — wertigen Metalle in diesen Bleicherden beträgt etwa 4:1.

| Reinigungsgang | Carbonylzahl [mg KOH/g] | Bromzahl [g/100 g] | Peroxidgehalt als $H_2O_2$ [ppm] |
|---|---|---|---|
| unbehandelt | 0,16 | 0,08 | 10 |
| 1 | <0,02 | <0,01 | 2 |
| 2 | <0,02 | 0,02 | 3 |
| 3 | 0,03 | 0,02 | 1 |
| 4 | <0,02 | 0,03 | 2 |
| 5 | 0,02 | 0,02 | <1 |
| 6 | 0,02 | 0,01 | 2 |

### Patentansprüche

1. Verfahren zur Reinigung von Tetrahydrofuran, *dadurch gekennzeichnet,* daß das Tetrahydrofuran mit einer Bleicherde behandelt wird.

2. Verfahren nach Anspruch 1, *dadurch gekennzeichnet,* daß Bleicherde mit einem Wassergehalt von weniger als 3 Gew.% verwendet wird.

3. Verfahren nach Anspruch 1, *dadurch gekennzeichnet,* daß man die Bleicherde in Mengen von 0,1 bis 5 Gew.%, bezogen auf das Tetrahydrofuran, anwendet.

4. Verfahren nach Anspruch 1, *dadurch gekennzeichnet,* daß man das Tetrahydrofuran bei Temperaturen zwischen Raumtemperatur und der Siedetemperatur des Tetrahydrofuran behandelt.

## Revendications

1. Procédé de purification du tétrahydrofuranne, caractérisé en ce que le tétrahydrofuranne est traité avec une terre décolorante.

2. Procédé suivant la revendication 1, caractérisé en ce que la terre décolorante employée possède une teneur en eau inférieure à 3% en poids.

3. Procédé suivant la revendication 1, caractérisé en ce que la terre décolorante est mise en oevre en une proportion comprise entre 0,1 et 5% du poids du tétrahydrofuranne.

4. Procédé suivant la revendication 1, caractérisé en ce que le traitement du tétra-hydrofuranne est effectué à une température comprise entre la température ordinaire et la température d'ébullition du tétra-hydrofuranne.

## Claims

1. A process for purifying tetrahydrofuran, *characterized in that* the tetrahydrofuran is treated with a bleaching earth.

2. A process as claimed in claim 1, *characterized in that* a bleaching earth containing less than 3% by weight of water is used.

3. A process as claimed in claim 1, *characterized in that* the amount of bleaching earth used is from 0.1 to 5% by weight, based on tetrahydrofuran.

4. A process as claimed in claim 1, *characterized in that* the tetrahydrofuran is treated at from room temperature to the boiling point of the tetrahydrofuran.